# EUROPEAN PATENT SPECIFICATION

(11) **EP 1 240 359 B1**
(45) Date of publication and mention of the grant of the patent: **19.11.2008**
(21) Application number: 00984206.3
(22) Date of filing: 11.12.2000
(51) Int. Cl.: C12Q 1/68, C12N 15/10

(54) **METHOD FOR SELECTIVELY ISOLATING A NUCLEIC ACID**
VERFAHREN ZUR SELEKTIVEN ISOLIERUNG EINER NUKLEINSÄURE
PROCEDE PERMETTANT D'ISOLER DE MANIERE SELECTIVE UN ACIDE NUCLEIQUE

(30) Priority: 10.12.1999 US 170140 P; 08.12.2000 US 733846
(43) Date of publication of application: 18.09.2002
(60) Divisional application: 07121191.6
(73) Proprietor: TRUSTEES OF PRINCETON UNIVERSITY, Princeton, NJ 08544-0086 (US); Generation Biotech, LLC, Lawrenceville NJ 08648 (US)
(72) Inventor: DAPPRICH, Johannes, Lawrenceville, NJ 08648 (US); CLEARY, Michele, A., West Windsor, NJ 08550 (US)
(74) Representative: Jump, Timothy John Simon
(86) International application number: PCT/US2000/033579
(87) International publication number: WO 2001/042510

(56) References cited:
- WO-A-00/56925
- WO-A-01/90419
- WO-A-94/01447
- US-A- 5 665 582
- TAGLE D A ET AL: "MAGNETIC BEAD CAPTURE OF EXPRESSED SEQUENCES ENCODED WITHIN LARGE GENOMIC SEGMENTS" NATURE, MACMILLAN JOURNALS LTD. LONDON, GB, vol. 361, no. 6414, 25 February 1993 (1993-02-25), pages 751-753, XP000380383 ISSN: 0028-0836 cited in the application

## Description

This invention relates to compositions and methods for separating polynucleotide molecules from a population of genomic DNA molecules.

One major area of current clinical research is the correlation of an individual's genetic profile to a susceptibility to disease and/or response to drug therapy. This area of research, which has been labeled pharmacogenomics, offers a strategy for targeting drugs to individuals, and for elucidating genetic predispositions and risks. In addition, pharmacogenomics provides for the possibility for an improved drug discovery process based on a better understanding of the molecular bases of complex diseases.

Identification of an individual's genetic profile can require the identification of particular nucleic acid sequences in the individual's genome. These particular nucleic acid sequences can include those that differ by one or a few nucleotides among individuals in the same species. For example, single-nucleotide polymorphisms (SNPs) are common variations in the DNA of individuals that are used to track inherited genetic patterns [1].

Current methods for identifying nucleic acid polymorphisms can be labor-intensive and expensive.

The invention is based in part on the discovery of a method for rapidly and economically isolating nucleic acid sequences containing particular nucleic acid sequences of interest. The invention provides a composition and method for sequence-specific extraction of polynucleotide sequences from a potentially complex mixture of genomic DNA molecules. One method of the invention, which is termed 'Allele-Specific Extraction (ASE), enables the distinction of two nearly identical sequences, for instance genes of maternal and paternal origin, by physical separation based on the identity of a heterozygous site. This ability, when coupled with standard methods commonly used for genotyping, permits rapid large-scale and cost-effective haplotyping of individuals, which can significantly reduce the size and decrease the duration of genetic profiling studies by focussing on the analysis of rare events, such as thereapeutic non-responders or adversely affected individuals [2].

In a first aspect of the invention, there is provided a method for separating a polynucleotide molecule from a population of genomic DNA molecules, the method comprising:
(a) providing a population of genomic DNA molecules comprising said polynucleotide molecule, wherein said polynucleotide molecule includes a first target nucleic acid sequence and a first distinguishing element;
(b) contacting said population of genomic DNA molecules with a first targeting element, wherein said first targeting element binds specifically to said first target nucleic acid sequence of said polynucleotide molecule;
(c) selectively attaching multiple separation groups to said bound first targeting element, wherein attachment of separation groups occurs only if said first targeting element is bound to said first target nucleic acid sequence, by extending said first targeting element so as to include multiple separation groups;
(d) immobilizing said attached separation group to a substrate, thereby forming an immobilized targeting element-separation group complex; and
(e) removing said immobilized targeting element-separation group complex from said population of genomic DNA molecules, thereby separating said polynucleotide molecule from said population of genomic DNA molecules;
wherein the first distinguishing element is a targeted single nucleotide polymorphism, the first targeting element is an oligonucleotide that partially overlaps the first distinguishing element, the first separation group is an immobilizable, non-terminating nucleotide, and the 3'-terminus of the oligonucleotide is complementary to the targeted single nucleotide polymorphism.

Preferred embodiments of the invention in any of its various aspects are as described below or as defined in the sub claims.

If desired, the population of genomic DNA molecules can be amplified using PCR or another amplification technique for the fragement(s) of interest prior to performing allele-specific extraction is the amount of available starting nucleic acid is insufficient for direct separation and subsequent analysis.

The targeting element is a molecule that binds specifically to a nucleic acid sequence in a population of genomic DNA molecules. In some embodiments, the targeting element is a nucleic acid, or nucleic acid derivative that hybridizes to a complementary target sequence in a population of genomic DNA molecules.

Examples of nucleic acid-based nucleic acid derivatives include, *e*.*g*., an oligonucleotide, oligo-peptide nucleic acid (PNA), oligo-LNA, or a ribozyme. The targeting element can alternatively be a polypeptide or polypeptide complex that binds specifically to a target sequence. Examples of polypeptide-based target elements include, *e.g*., a restriction enzyme, a transcription factor, RecA, nuclease, any sequence-specific DNA-binding protein. The targeting element can alternatively, or in addition be a hybrid, complex or tethered combination of one or more of these targeting elements.

In some embodiments, the targeting element binds to a target nucleic acid sequence in the vicinity of a discrete sequence known as a distinguishing element. A distinguishing element can include any sequence of interest. For example, the distinguishing element can be, e.g., a polymorphism (such as a single nucleotide polymorphism), a restriction site, a methylated restriction site, methylated sequence motif, secondary structure.

Association of a targeting element with a sequence of interest (such as one in the vicinity of a distinguishing element) can occur as part of a discrete chemical or physical association. For example, association can occur as part of, e.g., an enzymatic reaction, chemical reaction, physical association; polymerization, ligation, restriction cutting, cleavage, hybridization, recombination, crosslinking, pH-based cleavage.

The separation group can be any moiety that facilitates subsequent isolation and separation of an attached target element that is itself associated with a target nucleic acid. Preferred separation groups are those which can interact specifically with a cognate ligand. A preferred separation group is an immobilizable nucleotide, *e*.*g*., a biotinylated nucleotide or oligonucleotide. For example, separation groups can include biotin. Other examples of separation groups include, *e.g*., ligands, receptors, antibodies, haptens, enzymes, chemical groups recognizable by antibodies or aptamers.

The separation group can be immobilized on any desired substrate. Examples of desired substrates include, e.g., particles, beads, magnetic beads, optically trapped beads, microtiterplates, glass slides, papers, test strips, gels, other matrices, nitrocellulose, nylon. The substrate includes any binding partner capable of binding or crosslinking identical polynucleotide sequences via the separation element. For example, when the separation element is biotin, the substrate can include streptavidin.

The targeting element preferably includes (in whole or in part) a unique region located in proximity to the distinguishing element. The unique region uniquely identifies a polynucleotide sequence in conjunction with the particular region.

The targeting element binds to a target nucleic acid sequence partly overlapping with the distinguishing element.

In preferred embodiments, an enzyme-driven incorporation is performed of a separation element which becomes covalently attached to the targeting element (a specific oligonucleotide). The targeting element can itself be covalently attached or topologically linked to the targeted polynucleotide, which allows washing steps to be performed at very high stringency that results in reduced background and increased specificity.

For example, in preferred embodiments, the oligonucleotide has an extendable 3' hydroxyl terminus. When the targeting element is an oligonucleotide with an extendable 3' hydroxyl terminus and the separation group is an immobilizable nucleotide (such as a biotinylated nucleotide), the separation group is preferably attached to the targeting element by extending the oligonucleotide with a polymerase in the presence of the biotinylated nucleotide, thereby forming an extended oligonucleotide primer containing the immobilizable nucleotide.

If desired, the method can be repeated with second, third, or fourth or additional targeting elements by contacting the population of genomic DNA molecules with an additional targeting element (*e*.*g*., a second, third, fourth or more targeting element) that binds specifically to an additional nucleic acid sequence or sequences of interest in the population of genomic DNA molecules (which may be the same or different than the first nucleic acid of interest). A second (or additional) separation group is attached to the second targeting element. The attached second (or additional) separation group is attached to a substrate, thereby forming a second immobilized targeting element-separation group complex. The immobilized targeting element-separation group complex is then removed from the population of genomic DNA molecules, thereby separating the nucleic acid sequence of interest from the population of genomic DNA molecules.

Among the advantages of the invention is that it is directly compatible with standard genotyping methods and can be easily adapted for multiplexing. In addition, the method can be practiced in a bulk material and does not require single molecule dilution to achieve allele-specific separation. The method can be practiced as a single molecule technique, and the overall speed of the method is expected to be orders of magnitude faster than currently available processes. Moreover, the method does not involve live organisms such as rodents or yeast and thus eliminates any considerations and sources for error associated with such use. In addition, the method is suitable for robotic automation using commercially existing instrumentation for DNA extraction and purification. Moreover, the method allows for the allele-specific analysis of very long fragments of DNA.

The method is well-suited to identifying and isolating nucleic acids containing single nucleotide polymorphisms (SNPs). However, the method is not limited to the use of SNPs but also works with other genetic markers (for instance restriction sites, single tandem repeats, microsatellites), potentially including epigenetic patterns such as methylation. The method allows for the correlation of an unlimited number of sites constituting a haplotype *i*.*e*., is not limited to pairwise comparison of two selected sites. The method additionally allows for the generation of a re-usable library of genomic DNA. The library can be used to obtain haplotypes of previously untargeted genomic regions by regular genotype analysis without repeated allele-specific extraction.

In various embodiments, the methods disclosed herein are provided for manual operation in kit format, automated high-throughput operation, and/or in miniaturized & integrated format. The methods can be used in, e.g., human diseases, or predispositions to human diseases (including metabolic disease, cancer typing, diagnosis, and prognosis), analysis of organelle DNA (mitochondrial and chloroplast), plant traits, drug discovery, and in evolutionary studies, including tracking of disease evolution.

Unless otherwise defined, all technical and scientific terms used herein have the same meaning as commonly understood by one of ordinary skill in the art to which this invention belongs.

In addition, the materials, methods, and examples are illustrative only and not intended to be limiting.

Other features and advantages of the invention will be apparent from the following detailed description and claims.

### BRIEF DESCRIPTION OF THE DRAWINGS

FIG. 1 is a schematic illustration of a maternal and paternal chromosomal fragment containing several polymorphisms.
FIG. 2 is a schematic illustration showing annealing of oligonucleotides in the region of a polymorphic site.
FIG. 4 is a schematic illustration showing annealing of an oligonucleotide having a 3' base mismatch.
FIG. 5 is a schematic illustration showing elongation of an oligonucleotide lacking a mismatch.
FIG. 6 is a schematic illustration showing separation of a targeted fraction using solid support.
FIG. 7 is a graph showing attachment and release events of individual DNA molecules over time to a single bead as observed by a displacement sensor.
FIG. 8 is a graph showing attachment events of individual DNA molecules covalently linked to a separation element-bead complex.
FIG. 9 is a schematic illustration of multiple separation elements topologically locking a target fragment to a solid support.

The method provides for separating polynucleotide molecules from a population of genomic DNA molecules. The method allows for haplotyping through specific chromosomal fragment capture.

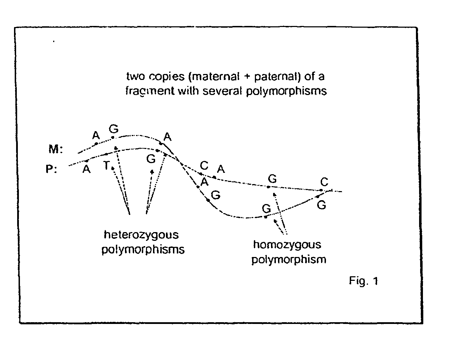

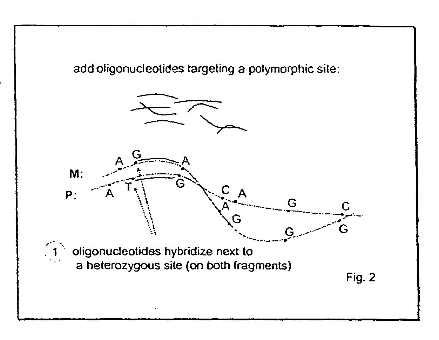

The method is divided into three steps:
1) "Targeting"
   In a first step, a targeting element uniquely distinguishing a particular polynucleotide sequence is targeted. FIG. 2 is a schematic illustration showing annealing of oligonucleotides to a polymorphic site.
2) "Distinction"
   In a second step, a process is carried out that distinguishes, based on the nature of the distinguishing element, between the targeted polynucleotide sequence(s) and any other sequence(s) present in the material by conditionally attaching or removing a functional group that can serve as a separation element for physical manipulation of the targeted polynucleotide sequence.
3) "Separation"
   In a third step, the targeted polynucleotide sequences are physically separated from the remainder of the sequences in a washing step after selective immobilization to a solid support via the attached separation element.
   In an exemplary embodiment, the method allows for separation of DNA fragments of maternal and paternal origin so that differences between the fragments can be assessed for the determination of a haplotype. The method can be practiced by manual operation and standard molecular biological equipment and materials as described below.
   If the sample is a combination of alleles from a heterozygous individual, there will be - by definition - locations that distinguish fragments containing the two different alleles. FIG. 1 is a schematic illustration of a maternal and paternal chromosomal fragment containing several polymorphisms, including heterozygous polymorphisms.

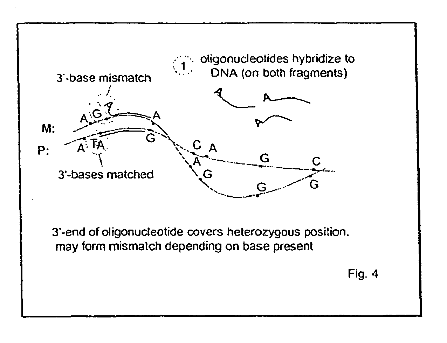

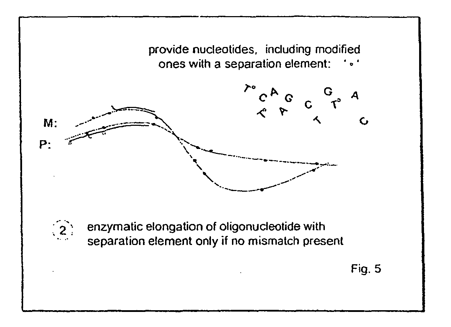

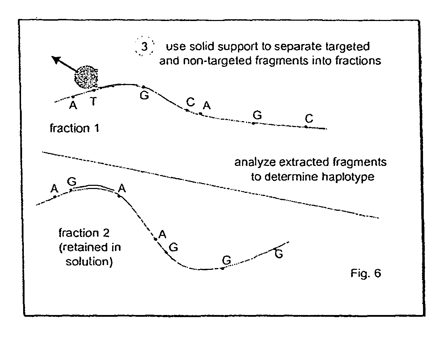

The steps are described in more detail below.
1) Targeting
   This step results in the recognition of a region within a polynucleotide sequence proximal to a site that allows distinction of specific polynucleotide fragments in a mixed population. This can be accomplished by use of an oligonucleotide (a targeting element) that hybridizes to a sequence partially overlapping a polymorphic site (a distinguishing element).
2) Distinction
   incorporation.
   The method allows the use of non-terminating nucleotides. This approach exploits the ability of a polymerase to recognize mismatched oligonucleotides - rather than mismatched individual nucleotides - to accomplish the distinction between targeted and non-targeted fragments: In this case an oligonucleotide is chosen such that it partially overlaps a polymorphic site during hybridization to the fragments, with the mismatch preferentially located at or near the 3'-end of the oligonucleotide, which is the location of enzymatic activity during elongation. Annealing of an oligonucleotide to complementary and mismatched target sequences is shown in FIG. 4.
   The oligonucleotide thus only gets elongated if the entire oligonucleotide hybridizes to the fragment (FIG. 5). Conditions can be chosen such that hybridization of a perfectly matched oligonucleotide is highly favored over hybridization of the same oligonucleotide to any site containing a mismatch [3] and if the oligonucleotide-fragment complex does not contain a base-mismatch that prevents the enzyme from binding and initiating the polymerization [4]. If biotinylated nucleotides are present in the reaction, only elongated oligonucleotides bound to targeted fragments will obtain a separation element in the form of multiple incorporated biotins.
3) Separation
   In a final step the fragments are separated into fractions that contain the targeted fragment (for example of maternal origin) versus other, non-targeted fragments (for example of paternal origin). This is accomplished by immobilizing the targeted fragment to a solid support which contains a second element with an affinity for the separation element, for instance by immobilizing the biotinylated oligonucleotide-fragment complex to streptavidin-coated magnetic beads, before in a washing step the unbound fraction of the sample is isolated from the beads containing the targeted fraction, allowing for separate analysis of both fractions. Use of a solid support to separate target fragments is shown in FIG. 6.

### Automated and High-throughput operation

The invention can be practiced in a fully automated embodiment by use of standard robotic liquid handling and sample preparation systems. In particular, robotic systems are commercially available that utilize magnetic beads to perform the extraction of DNA from a sample in a way that closely resembles the manual operation of such protocols. The adaptation of the method to those systems and their integration into a fully automated process line is straightforward; it requires no modification of equipment other than programming the system.

### Miniaturization and Separation of Genomic DNA Fragments

Miniaturization of the method as well as the separation of long fragments of genomic DNA is desirable in order to examine potentially small samples of tissue, for instance in cancer diagnosis, typing, and prognosis, and to obtain information about polymorphisms located over large contiguous regions. Fragments as large as 1-2 Mbp have been extracted from cells and manipulated for gel electrophoresis. [5].

The method can be performed on a single-molecular level. As an example, individual optically trapped streptavidin-coated beads can be used to capture single or numerous targeted fragments and manipulate them for instance in a microstructure [6]. Targeted fragments can be transported to separate locations such as different chambers of a microstructure for further processing (for instance amplification or sequence analysis [7]) or removal from the microstructure). The original sample is conserved with the exception of the targeted fragments and can be re-used for subsequent extraction of different fragments.

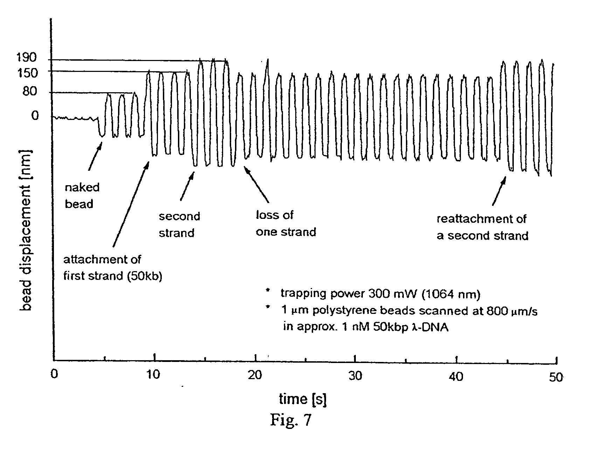

FIG. 7 illustrates repeated events of attachment (twice) and loss (once) of 50,000 base pair long DNA strands to a 1µm bead through hybridization of a biotinylated 16-mer oligonucleotide to the targeted fragment. Motion relative to the fluid exerts a force on the optically trapped bead, which can be measured in displacement on the vertical axis versus time. The oscillating pattern is the result of a back-and-forth motion of the optical trap which generates the displacement signal (see [6] for a detailed explanation). The significant events are changes in the envelope of the pattern, signaling attachment or occasional loss of individual molecules of DNA on the bead.

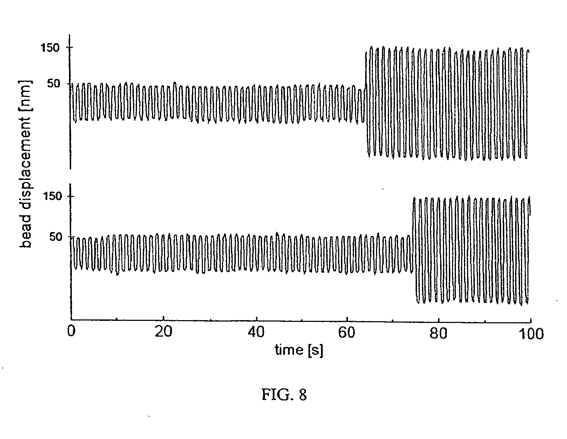

FIG. 8 illustrates two attachment events of a single DNA of 100,000 base pairs length to a 1 µm optically trapped bead. Losses of fragments are eliminated by ligating the biotinylated oligonucleotide to the targeted fragment; it is easily possible to work with long molecules of DNA for extended times. Direct fluorescent observation was used to confirm the attachment and observe the strand physically being removed from one region to another for storage or further manipulation.

A miniaturized and integrated device is a preferred platform in which the method can be practiced for instance for diagnostic purposes. This embodiment can readily be adapted to standard methods and devices for miniaturized, inexpensive and integrated genotyping and sequence analysis [8].

### Other Methods of Performing the Distinction Step

Not only polymerizing reactions as described above but more generally any distinguishing reaction that creates an allele-specific separation element enables the separation of targeted and non-targeted fragments. Many molecular biological as well as chemical methods exist or can be adapted to perform such a selective attachment or removal [9] of a separation element. For example, a population of genomic DNA molecules can be contacted with a targeting element attached to a separation group. The separation group is then removed from the bound targeting element. The remaining immobilizing groups are then immobilized on a substrate, forming an immobilized targeting element-separation group complex. The immobilized targeting element-separation group complex is then removed from the nucleic acid of interest, thereby separating said nucleic acid sequence of interest from said population of nucleic acid molecules.

### Binding of the Targeting Element to the Targeted Fragment

### a) Initial binding during targeting step

The targeting of polynucleotide fragments with sequence-specific oligonucleotides is straightforward when both are present in single-stranded form. A melting temperature can be calculated for each oligonucleotide-fragment complex below which hybridization occurs. It is possible to adjust the hybridization conditions (mainly temperature and salt / cation concentration) such that only perfectly matched oligonucleotides bind to the fragment. Considerable literature and protocols exist on the polymerase chain reaction (PCR), dye-terminator sequencing reactions as well as mini-sequencing or primer extension reactions, that are of similar nature as the enzymatic distinction reaction in this invention [10,11].

Single stranded DNA can be generated in several ways, for instance by heating and subsequent quenching on ice, NaOH denaturation or physical separation based on biotinylated PCR-primers that get incorporated into only one copy of a PCR product [10,12].

If double-stranded DNA is used as a template, such as genomic or plasmid DNA, the targeted location has to be rendered accessible in order for the oligonucleotide to bind to the fragment. This can be accomplished by heating the sample to a temperature at which the DNA begins to melt and form loops of single-stranded DNA.

Under annealing conditions and typically in an excess of oligonucleotide relative to template, the oligonucleotides will - due to mass action as well as their usually smaller size and thus higher diffusion coefficient - bind to homologous regions before renaturation of the melted fragment strands occurs. Oligonucleotides are also able to enter double-stranded fragments at homologous locations under physiological conditions (37 °C) [13].

This is relevant since the possibility of cross-hybridization between opposite strands of different alleles can lead to the extraction of a mismatched double-stranded hybrid of two alleles. It is usually undesirable to generate fully single-stranded template DNA due to this reason, although a robust link of the separation element and the targeted fragment - as discussed below - is able to retain the targeted fragment even under harsh denaturation and washing conditions.

Methods and kits have been developed to facilitate the sequence-specific introduction of oligonucleotides into double-stranded targets such as genomic or plasmid DNA [13,14]. A coating of oligonucleotides with DNA-binding proteins such RecA (*E*. *coli* recombination protein "A") or staphylococcal nuclease speeds up their incorporation several orders of magnitude compared to the introduction of analogous unmodified oligonucleotides at higher concentration and significantly increases the stability of such complexes [15], while still permitting enzymatic elongation of the introduced oligonucleotide [13].

### b) Binding during the separation step

It is possible to immobilize or otherwise capture very large molecules and complexes by a single separation element [6,14,16]. If mere hybridization between homologous regions is utilized, the length of the oligonucleotide-separation element has to be chosen of sufficient size to prevent a loss of the fragment during manipulation. For fragments of several hundred to thousand bases size relatively short oligonucleotides (20 bases) are sufficient, whereas longer fragment molecules will require oligonucleotides that bind over larger distances. It is important to note in this context that under conditions of manipulating fragments relative to the surrounding solution by means of an oligonucleotide-separation element the stability of hybridization is somewhat reduced, since temporary melting due to thermal fluctuations will occur on parts of the sequence that may lead to strand dissociation of a complex that is stable if there is no relative motion between components of the solution.

Another method for increasing the stability of the oligonucleotide-separation element-fragment complex is to provide a targeting element with the separation element already attached and further stabilize the binding in the distinction reaction. As an example, an oligonucleotide with biotinylated nucleotides incorporated during synthesis is elongated at its 3'-end with regular nucleotides (i.e. not containing biotin) over a significant distance after it has hybridized with the homologous region on the target fragment.

The enzymatic distinction between targeted and non-targeted fragments based on the identity of the targeted polymorphic site is achieved as discussed above before separation is achieved under conditions that facilitate hybridization of greatly elongated oligonucleotides to the targeted fragment and dissociation of short, unextended oligonucleotides from non-targeted fragments. This mode enables the use of oligonucleotides of relatively short length and converts them into tightly binding separation elements once they have been elongated after hybridization to the target fragment.

It is advantageous if a covalently or topologically linked bond is formed (or cleaved) between the separation element and the targeted fragment as a result of the distinguishing reaction. The former can be achieved by providing a reactive group linked to the separation element, so that upon selective incorporation of the separation element the reactive group is irreversibly attached to targeted fragments only. Examples for reactions that can be used for this purpose are described for instance in [17,18,19]. Examples for the formation of topologically linked bonds are described in [20].

### Binding of the targeted fragment to the solid support

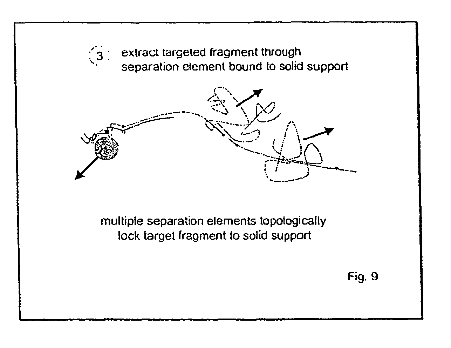

In the example discussed above, in which a regular oligonucleotide (not containing biotin) is elongated by use of non-terminating biotinylated nucleotides, a particularly strong attachment is formed by multiple binding events between multiple separation elements (i.e. biotins) and solid support (i.e. streptavidin-coated beads). It is significant that the elongation of the oligonucleotide produces numerous separation elements located over a potentially long distance of the targeted fragment. This is shown schematically in FIGs. 5 and 9.

Due to the twisted helical structure of double-stranded DNA, this complex binds to a for instance streptavidin-coated surface in a way that topologically links the targeted fragment to the solid support, provided the distance of the elongated region is significantly greater than the average distance between incorporated biotinylated nucleotides and the pitch of the helix (about 3.4 nm or ten basepairs per turn).

In a related version of the method, topologically improved binding of the targeted fragment to the solid support is achieved by use of multiple targeting and separation elements that simultaneously bind the fragment to a solid support with intervening sequences in between each element pair. It is necessary that such multiple targeting elements co-identify the targeted fragment so as to prevent binding of any of such elements to non-targeted fragments.

In preparation for the separation step it is advantageous to achieve fast on-rates as well as high selectivity and efficiency of binding between targeted fragments and solid support. If small fragments are used, it is sufficient to carry out the binding step by incubation on a rotator at room temperature. In the case of increasingly large fragments, two factors will interfere with the reaction and result in slower and less efficient binding:
a) increasingly large fragments have a significantly reduced diffusion coefficient
b) if only one separation element is present on the fragment, other regions of the same fragment may interfere with the binding step by effectively shielding the separation element from getting into sufficiently close proximity to the solid support to initiate the binding reaction
Relative motion between the targeted fragments and the solid support overcomes both problems. This can be achieved by different means, for instance by moving beads used for capturing back and forth through the solution by repeated precipitation and resuspension, or by electrophoretically generated movement.

### Non-specific binding to the solid support

Any non-specific binding of non-targeted fragments to the solid support may result in incomplete separation of targeted and non-targeted fragments. Especially single-stranded DNA may readily bind to untreated magnetic beads or other surfaces. The problem is overcome by exposing the surface to a solution containing components that saturate unspecific binding sites on the surface but do not interfere with the specific binding of the separation element [21].
As an example, a blocking buffer "MBSB" is used to suppress unspecific binding to beads (2.8 µm magnetic beads 'Dynabeads M-280 Streptavidin', Dynal A.S., Oslo, Norway, or 1 µm polystyrene beads ('Streptavidin Coated Latex'), Interfacial Dynamics Corporation, Portland, OR) with the result that biotinylated fragments are readily amplified by PCR compared to undetectable levels of product of non-biotinylated fragments on both types of beads (magnetic or polystyrene).

Buffer 'MBSA' is a solution containing 10 mM Tris pH 7.5, 2 mM EDTA, 0.2% Tween-20, 1M NaCl, 5 µg/ml BSA, 1.25 mg/ml 'carnation' dried milk (Nestle), 1 mg/ml glycine.
Buffer 'MBSB' is identical to 'MBSB' with the addition of 200 ng/µl sheared salmon sperm DNA (GIBCO BRL), average size ≈ 1000 basepairs, boiled for 3 min. and quenched on ice, and 50 nM each of oligonucleotides of the sequences TTAGTGCTGAACAAGTAGATCAGA (SEQ ID NO:3) and GTATATTCCAAGATCCATTAGCAG (SEQ ID NO:4).

Beads are washed twice in 1 ml "MBSA" by briefly vortexing and precipitating. Precipitation is performed with a particle collection magnet (Polysciences, Warrington, PA) for 1 min. (magnetic beads), or by centrifugation at 13,000 rpm on a table-top centrifuge for 3 min. (polystyrene beads). The beads are then incubated in 100 µl "MBSB" in a fresh tube rotating at RT for 2 hours and stored refrigerated in "MBSB".

Biotinylated and non-biotinylated fragments of identical sequence and 225 basepairs length were generated by PCR amplification of a region in the HLA (human leukocyte associated) locus.

An alternative to prevent contamination with unspecifically extracted, non-targeted fragments is the use of a cleavable linker, which enables the selective release of targeted fragments into solution after separation has been completed [22].

### Multiplexing

The method can be performed in a multiplexed fashion by targeting more than one fragment or more than one region on a fragment at once. As an example, this can be accomplished by use of multiple oligonucleotides of different sequence that target different polymorphisms.

### Generation of a haplotyping library

The method can be used to separate DNA (originating from chromosomal fragments of a sample containing multiple alleles) into fractions that contain the separated alleles only, and overlapping heterozygous regions of different fragments can be used to assemble information on coinherited genomic regions spanning contiguous fragments. Such a library can repeatedly be analyzed at different regions to study polymorphisms that were not classified previously, without the need for further separation of alleles.

### References Cited

[1] The use of single-nucleotide polymorphism maps in pharmacogenomics. McCarthy JJ, Hilfiker R. Nat Biotechnol. 2000 May;18(5):505-8. Review, and: Enthusiasm mixed with scepticism about single-nucleotide polymorphism markers for dissecting complex disorders. Syvanen AC, Landegren U, Isaksson A, Gyllensten U, Brookes A. First International SNP Meeting at Skokloster, Sweden, August 1998. Eur J Hum Genet. 1999 Jan;7(1):98-101.
[2] Research suggests importance of haplotypes over SNPs. Nat Biotechnol. 2000 Nov; 18:1134-5. and: Complex promoter and coding region beta 2-adrenergic receptor haplotypes alter receptor expression and predict in vivo responsiveness. Drysdale CM, McGraw DW, Stack CB, Stephens JC, Judson RS, Nandabalan K, Arnold K, Ruano G, Liggett SB. Proc Natl Acad Sci U S A. 2000 Sep 12;97(19):10483-8. and: Descent graphs in pedigree analysis: applications to haplotyping, location scores, and marker-sharing statistics. Sobel E, Lange K. Am J Hum Genet. 1996 Jun;58(6):1323-37. and: Loss of information due to ambiguous haplotyping of SNPs. Hodge SE, Boehnke M, Spence MA. Nat Genet. 1999 Apr;21(4):360-1. and: The predictive power of haplotypes in clinical response. Judson R, Stephens JC, Windemuth A. Pharmacogenomics 2000; (1)1-12. and: The accuracy of statistical methods for estimation of haplotype frequencies: an example from the CD4 locus. Tishkoff SA, Pakstis AJ, Ruano G, Kidd KK Am J Hum Genet 2000 Aug;67(2):518-22. and: A long-range regulatory element of Hoxc8 identified by using the pClasper vector. Bradshaw MS, Shashikant CS, Belting HG, Bollekens JA, Ruddle FH. Proc Nat1 Acad Sci U S A. 1996 Mar 19;93(6):2426-30. and: A new vector for recombination-based cloning of large DNA fragments from yeast artificial chromosomes. Bradshaw MS, Bollekens JA, Ruddle FH. Nucleic Acids Res. 1995 Dec 11;23(23):4850-6. and: Conversion of diploidy to haploidy. Nature, Vol.403, 17 Feb. 2000, p.723-4. and: Haplotype of multiple polymorphisms resolved by enzymatic amplification of single DNA molecules. Ruano G, Kidd KK, Stephens JC. Haplotype of multiple polymorphisms resolved by enzymatic amplification of single DNA molecules. Proc Nat1 Acad Sci U S A. 1990 Aug;87(16):6296-300.
[3] Direct haplotyping of kilobase-size DNA using carbon nanotube probes. Woolley AT, Guillemette C, Li Cheung C, Housman DE, Lieber CM. Nat Biotechnol. 2000 Jul;18(7):760-3.
[4] Proofreading DNA: recognition of aberrant DNA termini by the Klenow fragment of DNA polymerase I. Carver TE Jr, Hochstrasser RA, Millar DP. Proc Natl Acad Sci U S A. 1994 Oct 25;91(22):10670-4.
[5] Purification and staining of intact yeast DNA chromosomes and real-time observation of their migration during gel electrophoresis. Gurrieri S, Bustamante C. Biochem J. 1997 Aug 15;326 (Pt 1):131-8.
[6] DNA attachment to optically trapped beads in microstructures monitored by bead-displacement Dapprich J, Nicklaus N, Bioimaging 1998 Mar;6(1):25-32
[7] In situ localized amplification and contact replication of many individual DNA molecules. Mitra RD, Church GM. Nucleic Acids Res. 1999 Dcc 15;27(24):e34. and: Solid phase DNA sequencing using the biotin-avidin system. Stahl S, Hultman T, Olsson A, Moks T, Uhlen M. Nucleic Acids Res. 1988 Apr 11;16(7):3025-38. and: Single-molecule DNA digestion by lambda-exonuclease. Dapprich J. Cytometry. 1999 Jul 1;36(3):163-8.
[8] Determination of ancestral alleles for human single-nucleotide polymorphisms using high-density oligonucleotide arrays. Hacia JG, Fan JB, Ryder O, Jin L, Edgemon K, Ghandour G, Mayer RA, Sun B, Hsie L, Robbins CM, Brody LC, Wang D, Lander ES, Lipshutz R, Fodor SP, Collins FS. Nat Genet. 1999 Jun;22(2):164-7. and: Use of silver staining to detect nucleic acids. Mitchell LG, Bodenteich A, Merril CR. Methods Mol Biol. 1996;58:97-103. and: Technote#303, Bangs Laboratories, Fishers, IN
[9] Non-PCR-dependent detection of the factor V Leiden mutation from genomic DNA using a homogeneous invader microtiter plate assay. Ryan D, Nuccie B, Arvan D. Mol Diagn. 1999 Jun;4(2):135-44.
[10] Molecular Cloning: A Laboratory Manual. Sambrook J, Fritsch EF, Maniatis T; Second Edition 1989; Cold Spring Harbor Laboratory Press, NY.
[11] AmpliTaq™ product sheet, Perkin Elmer / Roche, Branchburg, NJ, and references therein
[12] Affinity generation of single-stranded DNA for dideoxy sequencing following the polymerase chain reaction. Mitchell LG, Merril CR. Anal Biochem. 1989 May 1;178(2):239-42.
[13]. Accelerated hybridization of oligonucleotides to duplex DNA. Iyer M, Norton JC, Corey DR., J Biol Chem. 1995 Jun 16;270(24):14712-7. and references cited therein
[14] RecA-assisted rapid enrichment of specific clones from model DNA libraries. Teintze M, Arzimanoglou II, Lovelace CI, Xu ZJ, Rigas B. Biochem Biophys Res Commun. 1995 Jun 26;211(3):804-11. and: Ability of RecA protein to promote a search for rare sequences in duplex DNA. Honigberg SM, Rao BJ, Radding CM. Proc Natl Acad Sci U S A. 1986 Dec;83(24):9586-90. and: Rapid plasmid library screening using RecA-coated biotinylated probes. Rigas B, Welcher AA, Ward DC, Weissman SM. Proc Natl Acad Sci U S A. 1986 Dec;83(24):9591-5. and: Preparation of a differentially expressed, full-length cDNA expression library by RecA-mediated triple-strand formation with subtractively enriched cDNA fragments. Hakvoort TB, Spijkers JA, Vermeulen JL, Lamers WH. Nucleic Acids Res. 1996 Sep 1;24(17):3478-80. and: Enriched full-length cDNA expression library by RecA-mediated affinity capture. Hakvoort TB, Vermeulen JL, Lamers WH. Gene Cloning and Analysis by RT-PCR, Edited by Siebert P and Larrick J, Biotechniques Books 1998, Natick, MA. and: ClonCapture™ cDNA Selection Kit, Clontech, Palo Alto, CA. and: Selective enrichment of specific DNA, cDNA and RNA sequences using biotinylated probes, avidin and copper-chelate agarose. Welcher AA, Torres AR, Ward DC. Nucleic Acids Res. 1986 Dec 22;14(24):10027-44.
[15] Homologous pairing and topological linkage of DNA molecules by combined action of E. coli RecA protein and topoisomerase I. Cunningham RP, Wu AM, Shibata T, DasGupta C, Radding CM. Cell. 1981 Apr;24(1):213-23. and: DNA hybrids stabilized by heterologies. Belotserkovskii BP, Reddy G, Zarling DA. Biochemistry. 1999 Aug 17;38(33):10785-92. and: Targeting in linear DNA duplexes with two complementary probe strands for hybrid stability. Sena EP, Zarling DA. Nat Genet. 1993 Apr;3(4):365-72.
[16] Preparation of a differentially expressed, full-length cDNA expression library by RecA-mediated triple-strand formation with subtractively enriched cDNA fragments. Hakvoort TB, Spijkers JA, Vermeulen JL, Lamers WH. Nucleic Acids Res. 1996 Sep 1;24(17):3478-80. and: Magnetic bead capture of expressed sequences encoded within large genomic segments. Tagle DA, Swaroop M, Lovett M, Collins FS. Nature. 1993 Feb 25;361(6414):751-3.
[17] Sequence-specific labeling of superhelical DNA by triple helix formation and psoralen crosslinking. Pfannschmidt C, Schaper A, Heim G, Jovin TM, Langowski J. Nucleic Acids Res. 1996 May 1;24(9):1702-9. and: Psoralens as photoactive probes of nucleic acid structure and function: organic chemistry, photochemistry, and biochemistry. Cimino GD, Gamper HB, Isaacs ST, Hearst JE. Annu Rev Biochem. 1985;54:1151-93. Review, and: Sequence-specific photo-induced cross-linking of the two strands of double-helical DNA by a psoralen covalently linked to a triple helix-forming oligonucleotide. Takasugi M, Guendouz A, Chassignol M, Decout JL, Lhomme J, Thuong NT, Helene C. Proc Natl Acad Sci U S A. 1991 Jul 1;88(13):5602-6. and: A novel approach to introduce site-directed specific cross-links within RNA-protein complexes. Application to the Escherichia coli threonyl-tRNA synthetase/translational operator complex. Zenkova M, Ehresmann C, Caillet J, Springer M, Karpova G, Ehresmann B, Romby P. Eur J Biochem. 1995 Aug 1;231(3):726-35.
[18] Sequence-specific recognition and cleavage of duplex DNA via triple-helix formation by oligonucleotides covalently linked to a phenanthroline-copper chelate. Francois JC, Saison-Behmoaras T, Barbier C, Chassignol M, Thuong NT, Helene C. Proc Natl Acad Sci U S A. 1989 Dec;86(24):9702-6. and: Sequence-specific artificial photo-induced endonucleases based on triple helix-forming oligonucleotides. Perrouault L, Asseline U, Rivalle C, Thuong NT, Bisagni E, Giovannangeli C, Le Doan T, Helene C. Nature. 1990 Mar 22;344(6264):358-60. and: Recognition and photo-induced cleavage and cross-linking of nucleic acids by oligonucleotides covalently linked to ellipticine. Le Doan T, Perrouault L, Asseline U, Thuong NT, Rivalle C, Bisagni E, Helene C. Antisense Res Dev. 1991 Spring; 1(1):43-54. and: Unambiguous demonstration of triple-helix-directed gene modification. Barre FX, Ait-Si-Ali S, Giovannangeli C, Luis R, Robin P, Pritchard LL, Helene C, Harel-Bellan A. Proc Natl Acad Sci U S A. 2000 Mar 28;97(7):3084-8. and: Sequence-specific intercalating agents: intercalation at specific sequences on duplex DNA via major groove recognition by oligonucleotide-intercalator conjugates. Sun JS, Francois JC, Montenay-Garestier T, Saison-Behmoaras T, Roig V, Thuong NT, Helene C. Proc Natl Acad Sci U S A. 1989 Dec;86(23):9198-202.
[19] Strand specific cleavage ofphosphorothioate-containing DNA by reaction with restriction endonucleases in the presence of ethidium bromide. Sayers JR, Schmidt W, Wendler A, Eckstein F Nucleic Acids Res 1988 Feb 11;16(3):803-14. and: 5'-3' exonucleases in phosphorothioate-based oligonucleotide-directed mutagenesis. Sayers JR, Schmidt W, Eckstein F. Nucleic Acids Res. 1988 Feb 11;16(3):791-802.
[20] Padlock oligonucleotides for duplex DNA based on sequence-specific triple helix formation. Escude C, Garestier T, Helene C. Proc Natl Acad Sci U S A. 1999 Sep 14;96(19):10603-7. and: PCR-generated padlock probes detect single nucleotide variation in genomic DNA. Antson DO, Isaksson A, Landegren U, Nilsson M. Nucleic Acids Res. 2000 Jun 15;28(12):E58. and: Padlock probes reveal single-nucleotide differences, parent of origin and in situ distribution of centromeric sequences in human chromosomes 13 and 21. Nilsson M, Krejci K, Koch J, Kwiatkowski M, Gustavsson P, Landegren U. Nat Genet. 1997 Jul;16(3):252-5.
[21] Prevention of nonspecific binding of avidin. Duhamel RC, Whitehead JS. Methods Enzymol. 1990;184:201-7.
[22] Dynal product sheet for 'Dynabeads M-280 Streptavidin', Dynal A.S., Oslo, Norway; www.dynal.no, and references cited therein, and: Pierce Chemical Technical Library: "Avidin-biotin", Pierce, Rockford, IL; www.piercenet.com, and references cited therein. and: Affinity isolation of transcriptionally active murine erythroleukemia cell DNA using a biotinylated nucleotide analog. Dawson BA, Herman T, Lough J. J Biol Chem. 1989 Aug 5;264(22):12830-7.

## Claims

1. A method for separating a polynucleotide molecule from a population of genomic DNA molecules, the method comprising:
(a) providing a population of genomic DNA molecules comprising said polynucleotide molecule, wherein said polynucleotide molecule includes a first target nucleic acid sequence and a first distinguishing element;
(b) contacting said population of genomic DNA molecules with a first targeting element, wherein said first targeting element binds specifically to said first target nucleic acid sequence of said polynucleotide molecule;
(c) selectively attaching multiple separation groups to said bound first targeting element, wherein attachment of separation groups occurs only if said first targeting element is bound to said first target nucleic acid sequence, by extending said first targeting element so as to include multiple separation groups;
(d) immobilizing said attached separation group to a substrate, thereby forming an immobilized targeting element-separation group complex; and
(e) removing said immobilized targeting element-separation group complex from said population of genomic DNA molecules, thereby separating said polynucleotide molecule from said population of genomic DNA molecules;
wherein the first distinguishing element is a targeted single nucleotide polymorphism, the first targeting element is an oligonucleotide that partially overlaps the first distinguishing element, the first separation group is an immobilizable, non-terminating nucleotide, and the 3'-terminus of the oligonucleotide is complementary to the targeted single nucleotide polymorphism.

2. The method of claim 1, wherein separation groups are biotinylated nucleotides.

3. The method of claim 1, wherein said substrate is a particle, bead, magnetic bead, or glass surface.

4. The method of claim 1, wherein the immobilized targeting-element-separation group complex is topologically attached to the substrate via multiple separation groups.

5. The method of claim 1, wherein step (c) is performed by extending the 3'-terminus of the oligonucleotide in the presence of the immobilizable, non-terminating nucleotide by a DNA polymerase.

## Patentansprüche

1. Methode zum Trennen eines Polynucleotidmoleküls von einer Population genomischer DNA-Moleküle, wobei die Methode Folgendes umfasst:
(a) Bereitstellen einer Population genomischer DNA-Moleküle umfassend das Polynucleotidmolekül, wobei das Polynucleotidmolekül eine erste Targetnucleinsäuresequenz und ein erstes Unterscheidungselement umfasst;
(b) Kontaktieren der Population genomischer DNA-Moleküle mit einem ersten Targeting-Element, wobei das erste Targeting-Element sich spezifisch an die erste Targetnucleinsäuresequenz des Polynucleotidmoleküls bindet;
(c) selektives Anknüpfen mehrerer Trennungsgruppen an das gebundene erste Targeting-Element, wobei das Anknüpfen von Trennungsgruppen nur dann erfolgt, wenn das erste Targeting-Element an die erste Targetnucleinsäuresequenz angeknüpft ist, durch Verlängern des ersten Targeting-Elements, um mehrere Trennungsgruppen einzuschließen;
(d) Immobilisieren der angeknüpften Trennungsgruppe an ein Substrat unter Bildung eines immobilisierten Targeting-Element-Trennungsgruppenkomplexes; und
(e) Entfernen des immobilisierten Targeting-Element-Trennungsgruppenkomplexes von der Population genomischer DNA-Moleküle unter Trennung des Polynucleotidmoleküls von der Population genomischer DNA-Moleküle;
wobei das erste Unterscheidungselement ein angezielter Einzel-Nucleotid-Polymorphismus ist, wobei das erste Targeting-Element ein Oligonucleotid ist, der das erste Unterscheidungselement teilweise überlappt, wobei die erste Trennungsgruppe ein immobilisierbares, nichtterminierendes Nucleotid ist, und der 3'-Terminus des Oligonucleotids zum angezielten Einzel-Nucleotid-Polymorphismus komplementär ist.

2. Methode nach Anspruch 1, wobei die Trennungsgruppen biotinylierte Nucleotide sind.

3. Methode nach Anspruch 1, wobei das Substrat ein Teilchen, eine Perle, eine magnetische Perle oder eine Glasfläche ist.

4. Methode nach Anspruch 1, wobei der immobilisierte Targeting-Element-Trennungsgruppenkomplex topologisch durch multiple Trennungsgruppen an das Substrat angeknüpft ist.

5. Methode nach Anspruch 1, wobei Schritt (c) durch Verlängern des 3'-Terminus des Oligonucleotids in Gegenwart des immobilisierbaren, nichtterminierenden Nucleotids durch eine DNA-Polymerase durchgeführt wird.

## Revendications

1. Méthode de séparation d'une molécule polynucléotidique à partir d'une population de molécules d'ADN génomique, la méthode comprenant :
(a) l'obtention d'une population de molécules d'ADN génomique comprenant ladite molécule polynucléotidique, où ladite molécule polynucléotidique comporte une première séquence d'acides nucléiques cible et un premier élément distinctif ;
(b) la mise en contact de ladite population de molécules d'ADN génomique avec un premier élément de ciblage, où ledit premier élément de ciblage se lie spécifiquement à ladite première séquence d'acides nucléiques cible de ladite molécule polynucléotidique ;
(c) le greffage sélectif de groupes de séparation multiples au dit premier élément de ciblage lié, où le greffage des groupes de séparation a lieu uniquement si ledit premier élément de ciblage est lié à ladite première séquence d'acides nucléiques cible, par extension dudit premier élément de ciblage de façon à inclure des groupes de séparation multiples ;
(d) l'immobilisation dudit groupe de séparation greffé sur un substrat, formant ainsi un complexe élément de ciblage-groupe de séparation immobilisé ; et
(e) l'élimination dudit complexe élément de ciblage-groupe de séparation immobilisé de ladite population de molécules d'ADN génomique, produisant ainsi la séparation de ladite molécule polynucléotidique de ladite population de molécules d'ADN génomique ;
où le premier élément distinctif est le polymorphisme d'un seul nucléotidique visé, le premier élément de ciblage est un oligonucléotide qui chevauche en partie le premier élément distinctif, le premier groupe de séparation est un nucléotide non terminal immobilisable et l'extrémité 3'-terminale de l'oligonucléotide est complémentaire au polymorphisme d'un seul nucléotidique visé.

2. Méthode de la revendication 1, où les groupes de séparation sont des nucléotides biotinylés.

3. Méthode de la revendication 1, où ledit substrat est une particule, une bille, une bille magnétique ou une surface de verre.

4. Méthode de la revendication 1, où le complexe élément de ciblage-groupe de séparation immobilisé est greffé sur le plan topologique au substrat par l'intermédiaire de groupes de séparation multiples.

5. Méthode de la revendication 1, où l'étape (c) est effectuée par une extension de l'extrémité 3'-terminale de l'oligonucléotide par une ADN-polymérase en la présence du nucléotide non terminal immobilisable.
